# EUROPÄISCHE PATENTSCHRIFT

(11) **EP 1 062 986 B1**
(45) Veröffentlichungstag und Bekanntmachung des Hinweises auf die Patenterteilung: **05.04.2006**
(21) Anmeldenummer: 00250205.2
(22) Anmeldetag: 23.06.2000
(51) Int. Cl.: A61N 1/375, H01R 4/50

(54) **Implantierbares elektronisches Gerät mit Anschluss- und Verriegelungseinrichtung**
Implantable electronic device with connecting and locking devices
Appareil éléctronique implantable avec connecteur et verrouillage

(30) Priorität: 25.06.1999 DE 19930238
(43) Veröffentlichungstag der Anmeldung: 27.12.2000
(73) Patentinhaber: BIOTRONIK Mess- und Therapiegeräte GmbH & Co Ingenieurbüro Berlin, 12359 Berlin (DE)
(72) Erfinder: Lorkowski, Lars, 10117 Berlin (DE)
(74) Vertreter: Lindner-Vogt, Karin L.

(56) Entgegenhaltungen:
- WO-A-98/57702

## Beschreibung

Die Erfindung betrifft ein implantierbares elektronisches Gerät gemäß dem Oberbegriff des Anspruchs 1.

Aus der amerikanischen Patentschrift US-PS 4 540 236 ist eine automatische Verriegelungsvorrichtung für einen Stecker einer mit einem Herzschrittmacher zu verbindenden koaxialen Elektrodenleitung bekannt, bei welcher hakenförmige Halteklauen vorgesehen sind, die in eine an der Steckerspitze vorgesehene Ringnut eingreifen und dadurch eine Sicherung der Steckerverbindung gegen axiale Zugbelastungen bewirken. Um den Stecker aus der Anschlußvorrichtung entfemen zu können, ist eine manuelle Druckeinleitung von der Stirnseite des Headers erforderlich, um über ein Gestänge das Abspreizen der Halteklauen zu bewirken.

Weiterhin ist aus der amerikanischen Patentschrift US-PS 5 252 090 eine selbst verriegelnde Steckverbindung für den Stecker einer implantierbaren, koaxialen Elektrodenleitung bekannt, bei der in der Anschlußvorrichtung ein elektrisches Kontaktelement mit mehreren federnden, im wesentlichen radial nach innen weisenden Zungen vorgesehen sind, welche durch die Steckerspitze gegen die Federkraft radial nach außen gedrückt werden und eine Klemmkraft erzeugen, wenn der Stecker in die Anschlußeinrichtung geschoben wird. Das elektrische Kontaktelement weist zusätzliche, schräg nach außen gerichtete Federarme auf, durch welche der Ktemmkontakt der nach innen weisenden Zungen an der Steckerspitze aufgehoben werden kann, wenn diese Federarme von außen durch eine Druckkraft belastet werden.

Darüberhinaus ist gemäß der amerikanischen Patentschrift US-PS 5 697 804 ein Herzschrittmacher mit einer Verriegelungsvorrichtung bekannt, bei welcher das elektrische Kontaktelement als Spiralfeder ausgebildet ist. Durch eine Drehbewegung des Stecker der an den Herzschrittmachers anzuschließend Elektrodenleitung innerhalb der Anschlußvorrichtung ist der Durchmesser der Spiralfeder soweit verringerbar, daß eine, Klemmwirkung zwischen den einzelnen Windungen der Spiralfeder und der Mantelfläche des differenten Pol des Steckers erzeugt wird.

Die bekannten, relativ kompliziert aufgebauten Verriegelungseinrichtungen weisen den Nachteil auf, daß sie bei der Bedienung, insbesondere beim Lösen der Verriegelung, sehr schwer zu handhaben sind, da die Verriegelungseinrichtungen nur durch eine frontal oder seitlich von der Headerwandung manuell einzuleitende Druckkraft gelöst werden können. Darüberhinaus ist in nachteiliger Weise nicht erkennbar, ob der in der Anschlußvorrichtung befindliche Stecker auch wirklich verriegelt worden ist.

Letztendlich ist aus der internationalen Patentanmeldung WO98/57702 ein schraubfreies Anschlußsystem für mindestens eine Elektrodenleitung an einen Herzschrittmacher bekannt. An dem Header des Herzschrittmachers ist eine Verriegelungseinrichtung vorgesehen, welche einen mit einer abgesetzten Welle verbundener Handhebel zum Verriegeln einer angeschlossenen Elektrodenleitung aufweist. Die durch den Handhebel verdrehbare Welle erstreckt sich quer zur Achse der für das Einstecken der Elektrodenleitung vorgesehenen Bohrung(en) und weist an ihrem Umfang verteilt sattelfürmige Ausnehmungen unterschiedlicher Tiefe auf, welche sich paarweise und orthogonal gegenüberliegen. In Offenstellung der Verriegelungseinrichtung bilden die großen Ausnehmungen der verdrehbaren Welle einen Teil der Bohrungswandung und liegen an der Umhüllung der Elektrodenleitung(en) an. Durch Schwenken des Handhebels gelangen die kleineren Ausnehmungen der Welle unter Verringerung des freien Querschnitts der Bohrung mit der Umhüllung der Elektrodenleitung(en) in Wirkkontakt und blockieren die Elektrodenleitung(en) durch die Klemmwirkung gegen ein axiale Bewegung.

Diese Verriegelungseinrichtung ist als kompliziertes Formteil ausgebildet und weist den weiteren Nachteil auf, daß die Klemmwirkung der Welle zum Halten des oder der Stecker(s) auf den Bereich der Leitungsisolation einer Elektrodenleitung beschränkt ist. Dadurch erfolgt im wesentlichen nur eine mechanische Zugentlastung der Elektrodenleitung, ohne daß die eigentlichen Kontaktstellen zwangsläufig gesichert sein müssen. Somit ist keine Aussage über die Qualität der Kontaktierung möglich, falls der Stecker der Elektrodenleitung den für ihn vorgesehene Kontaktbereich nach dem Einstecken beispielsweise nicht oder nur teilweise erreicht. Darüberhinaus sind in nachteiliger Weise Beschädigungen der Leitungsstränge bzw. ein Kurzschluß zwischen einzelnen Leitungssträngen nicht mit Sicherheit auszuschließen.

Ausgehend von den Mängeln des Standes der Technik liegt der Erfindung deshalb die Aufgabe zugrunde, ein implantierbares elektronisches Gerät der eingangs genannten Gattung mit einer Verriegelungseinrichtung für den Stecker einer mit dem Gerät verbindbaren, koaxialen Elektrodenleitung anzugeben, durch welche mit einfachen Mitteln ein Fixation des eingesteckten Steckers bei einer auf die Elekrodenleitung wirkenden, im wesentlichen axial gerichteten, Zugbelastung erreichbar ist und gleichzeitig mit Sicherheit eine gute Kontaktierung erfolgt.

Die Aufgabe wird durch die im Anspruch 1 angegebenen Merkmale gelöst.

Die Erfindung schließt die Erkenntnis ein, daß bei Einsatz eines exzentrischen, durch einen Hebel eines manuell aktivierbaren Kurbeltriebs aufgrund der Hebelwirkung bereits bei einer relativ geringen Schwenkbewegung durch den Exzenter eine große Kraftwirkung erreichbar ist, welche bevorzugt zur Erzeugung von Klemmkräften zwischen zwei Körpern genutzt werden kann. Durch Wahl der Exzentrizität und die Form des Exzenters ist eine bequeme Dosierung der erzeugten Klemmkraft in Abhängigkeit von der Größe des Schwenkwinkels möglich.

Erfindungsgemäß weist die im Header eines implantierbaren elektronischen Geräts, bevorzugt eines Herzschrittmachers, angeordnete, im wesentlichen buchsenförmige Anschlußvorrichtung für den Stecker einer koaxiale Elektrodenleitung eine Verriegelungseinrichtung auf, welche aus einer manuell betätigbaren, exzentrisch gelagerten Spannocke und einem in der Anschlußvorrichtung vorgesehenen Kontaktelement für den differenten Pol des Steckers einer mit dem Herzschrittmacher zu verbindenden koaxialen Elektrodenleitung gebildet ist.

Die exzentrisch gelagerte Spannocke steht an ihrem Rand mit dem Kontaktelement in Wirkungseingriff, so daß eine Drehbewegung der Spannocke auf das Kontaktelement übertragen wird. Dadurch wird das den differenten Pol des Steckers formschlüssig umgreifende Kontaktelement derart reversibel verformt, daß zwischen dem differenten Pol und dem Kontaktelement eine kraftschlüssige Verbindung entsteht, welche den Stecker innerhalb der Anschlußvorrichtung fixiert und gleichzeitig einen verbesserten galvanischen Kontakt zwischen dem Kontaktelement und dem differentenn Pol des Steckers sichert.

Entsprechend der bevorzugten Ausführungsform der Erfindung ist das Kontaktelement an der Steckeraufnahme der Anschlußvorrichtung durch Schweißen befestigt. Das aus einem Federmaterial bestehende Kontaktelement ist zungenförmig ausgebildet und erstreckt sich im wesentlichen quer zur Längsachse der Anschlußvorrichtung. Es weist eine Bohrung auf, durch welche der differente Pol des in der Anschlußvorrichtung befindlichen Steckers geführt ist.

Nach der bevorzugten Ausführungsform der Erfindung ist die exentrisch gelagerte Spannocke im wesentlichen scheibenförmig ausgebildet, unter Verwendung eines Lagerzapfens im Header gelagert und mit dem an der Seitenwandung des Headers angeordneten Handhebel zum Erzeugen einer Drehbewegung verbunden.

Die Drehachse der exzentrisch gelagerten Spannocke erstreckt sich im wesentlichen senkrecht zur Achse des Steckers des Elektrodenleitung, so daß sich das freie Ende des Kontaktelements auf dem Rand der exzentrisch gelagerten Spannocke abstützt. Da sich der Abstand des Randes in Bezug auf die Drehachse der Spannocke beim Verdrehen ändert, ist das federnd ausgebildete Kontaktelement durch eine mittels Schwenken des Handhebels erzeugte Drehbewegung der Spannocke reversibel verformbar und wird dabei relativ zu dem differenten Pol des Steckers unter Herstellung einer kraftschlüssigen Verbindung, insbesondere einer Klemmverbindung, mit diesem bewegt.

Diese kraftschlüssige Verbindung entsteht auf einfache Weise dadurch, daß sich der durch Projektion auf die Querschnittsfläche des differenten Pols bestimmte, freie Durchmesser der in dem Kontaktelement befindlichen Bohrung verringert. Dies bewirkt durch Klemmwirkung bereits bei relativ geringer Auslenkung des Kontaktelements eine ausreichend große Haltekraft zwischen differentem Pol und dem Kontaktelement zum Fixieren des Steckers bei einer axialen Zugbelastung der koaxialen Elektrodenleitung.

Entsprechend der bevorzugten Ausführungsform der Erfindung weist die exzentrisch gelagerte Spannocke im wesentlichen die Form einer Kreisscheibe auf, wodurch die Erzeugung einer besonders gleichförmigen Klemmkraft ermöglicht wird. Gleichzeitig ist durch die Verwendung des Handhebels der Kraftaufwand für die Erzeugung der zum Fixieren des Steckers erforderlichen Klemmkraft relativ gering.

Somit kann die Verriegelung des Steckers einer koaxialen Elektrodenleitung in einem zu implantierenden Herzschrittmacher sehr bequem gehandhabt werden, was beispielsweise unter Operationsbedingungen von einem besonderen Vorteil ist.

In einer vorteilhaften Weiterbildung der Erfindung ist vorgesehen, daß das Kontaktelement zum Erzeugen der Klemmwirkung durch reversibles Verformen mittels der exzentrisch gelagerten Spannocke aus einem entspannten Zustand in einen gespannten Zustand überführt wird.

Da für die Spannocke entsprechend einer Variante der Erfindung ein Drehbereich von bevorzugt 90° vorgesehen ist, befindet sich der zum Bedienen der Verriegelungseinrichtung an der Außenseite des Headers angeordnete, mit der Spannocke verbundene Handhebel in einer zur Achse der Anschlußvorrichtung im wesentlichen senkrechten Position, wenn die Verriegelungseinrichtung geöffnet wird. Bei geschlossener Verriegelung erstreckt sich der Handhebel im wesentlichen parallel zur Achse der Anschlußvorrichtung und paßt sich dadurch in einer, insbesondere für die Implantation des Herzschrittmachers, vorteilhaften Weise an die Form des Headers an.

Aus der Stellung des Handhebels ist somit auf einfache Weise der jeweilige Verriegelungszustand zu erkennen, was für die praktische Anwendung der erfindungsgemäßen Verriegelungseinrichtung von zusätzlichem Vorteil ist.

Entsprechend einer anderen vorteilhaften Weiterbildung der Erfindung ist an der Außenseite des Headers und der dem Header zugewandten Seite des freien Endes des Handhebels jeweils ein Rastmittel vorgesehen, welche sich bei geschlossener Verriegelungseinrichtung kraft-und/oder formschlüssig in Eingriff befinden und den Handhebel gegen eine unerwünschte, zum Öffnen der Verriegelungseinrichtung führende Rückbewegung fixieren. Die Rastmittel sind als Ansatz und eine den Ansatz aufnehmende Auskehlung entsprechender Größe ausgebildet.

Nach einer zusätzlichen Variante der Erfindung ist das Kontaktelement zweiteilig ausgebildet, wobei in jedem der Teilstücke eine Bohrung zur Aufnahme des differenten Pols eines jeweils in einer Anschlußvorrichtung befindlichen Steckers vorgesehen ist. Dadurch ist es auf einfache Weise möglich, mit einer exzentrisch gelagerten Spannocke gleichzeitig für die Stecker zweier Elektrodenleitungen jeweils eine Verriegelungseinrichtung zu bilden.

Die beiden Teilstücke des Kontaktelements sind durch einen Verbinder aus Kunststoff miteinander gekoppelt, da die Stecker der einzelnen koaxialen Elektrodenleitungen ein unterschiedliches Potential führen und ein Potentialausgleich vermieden werden muß.

Entsprechend einer anderen günstigen Ausführungsform der Erfindung ist die exzentrisch gelagerte Spannocke zwischen zwei im wesentlichen gleichartig ausgebildeten, vertikal übereinander positionierten Anschlußvorrichtungen angeordnet. Die an den Anschlußvorrichtungen vorgesehenen Kontaktelemente sind gleichartig ausgebildet und sich stützen jeweils bei im wesentlichen spiegelsymmetrischer Anordnung zwecks Bildung einer Verriegelungsvorrichtung auf dem Spannocke ab. Bei einer über den Handhebel erzeugten Drehbewegung der exzentrisch gelagerten Spannocke führen die Kontaktelemente gleichzeitig eine Schwenkbewegung mit entgegengesetztem Richtungssinn aus, so daß durch eine Handhebelbewegung an beiden differenten Polen die zum Fixieren des entsprechenden Steckers erforderliche Klemmkraft erzeugt werden kann.

Eine gleichermaßen vorteilhafte Betätigung von zwei Verriegelungseinrichtungen ist nach einer zusätzlichen Ausführungsform der Erfindung erreichbar, wenn für jede Anschlußvorrichtung eine gesonderte, jeweils exzentrisch gelagerte, Spannocke vorgesehen ist, wobei die erste Spannocke eine Verbindung mit einem Handhebel aufweist und die Drehbewegung der ersten Spannocke von diesem kraft- und formschlüssig auf die zweite Spannocke übertragen wird. Zur bequemen Übertragung der Drehbewegung weisen die beiden exzentrisch gelagerten Spannocken jeweils einen Lagerzapfen auf, welcher, zumindest an einem Abschnitt ihrer Peripherie, eine Außenverzahnung trägt.

Nach einer Variante der Erfindung ist zur Übertragung der Drehbewegung eine mittelbare Verbindung zwischen den Spannocken vorgesehen, welche sich bevorzugt durch ein Ritzel realisierbar ist, welches die Lagerzapfen der Spannocken unter Bildung eines Getriebes miteinander verbindet.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1a: eine bevorzugtes Ausführungsform der Erfindung mit geöffneter Verriegelungseinrichtung in einer perspektivischen Seitenansicht,
- Figur 1b: eine Teilansicht der in Figur 1a gezeigten Ausführungsform der Erfindung bei geöffnetem Header in perspektivischer Darstellung,
- Figur 1c: die Darstellung der Ansicht eines Schnittes längs der Linie A...A gemäß Figur 1b,
- Figur 1d: die Darstellung der Ansicht eines Schnittes längs der Linie B...B gemäß Figur 1c,
- Figur 2a: die in Figur 1a gezeigte Ausführungsform der Erfindung mit geschlossener Verriegelungseinrichtung in einer perspektivischen Sei- tenansicht,
- Figur 2b: eine Teilansicht der in Figur 2a gezeigten Ausführungsform der Erfindung bei geöffnetem Header in perspektivischer Darstellung,
- Figur 2c: die Darstellung der Ansicht eines Schnittes längs der Linie C...C gemäß Figur 2b,
- Figur 2d: die Darstellung der Ansicht eines Schnittes längs der Linie D...D gemäß Figur 2c,
- Figur 3: eine günstige Weiterbildung der Erfindung,
- Figur 4: eine andere vorteilhafte Ausführungsform der Erfindung in Darstellung eines Teil-Längsschnittes durch eine Anschlußvorrichtung, sowie
- Figur 5: eine vorteilhafte Variante der in Figur 4 gezeigten Ausführungsform der Erfindung in Darstellung eines Teil-Längsschnittes.

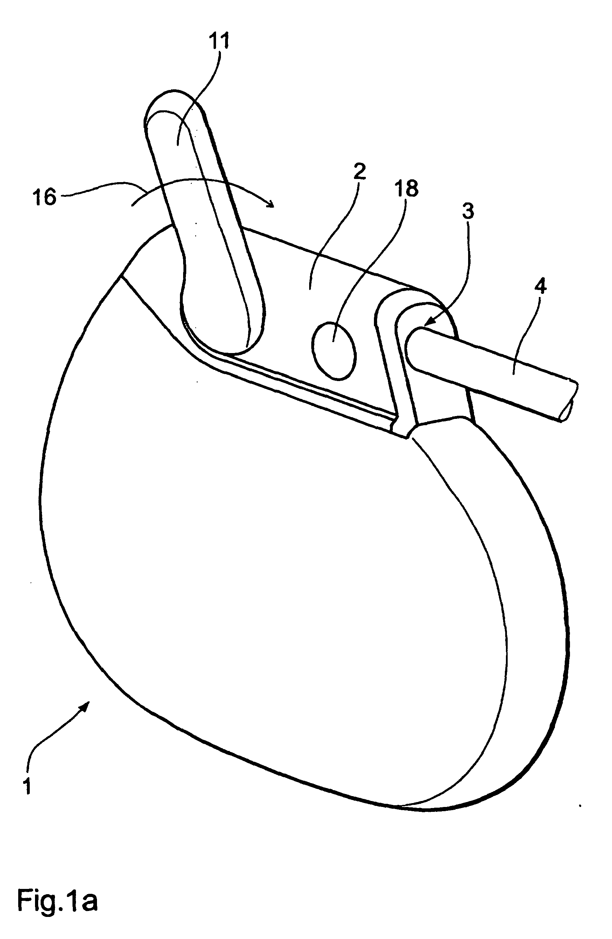

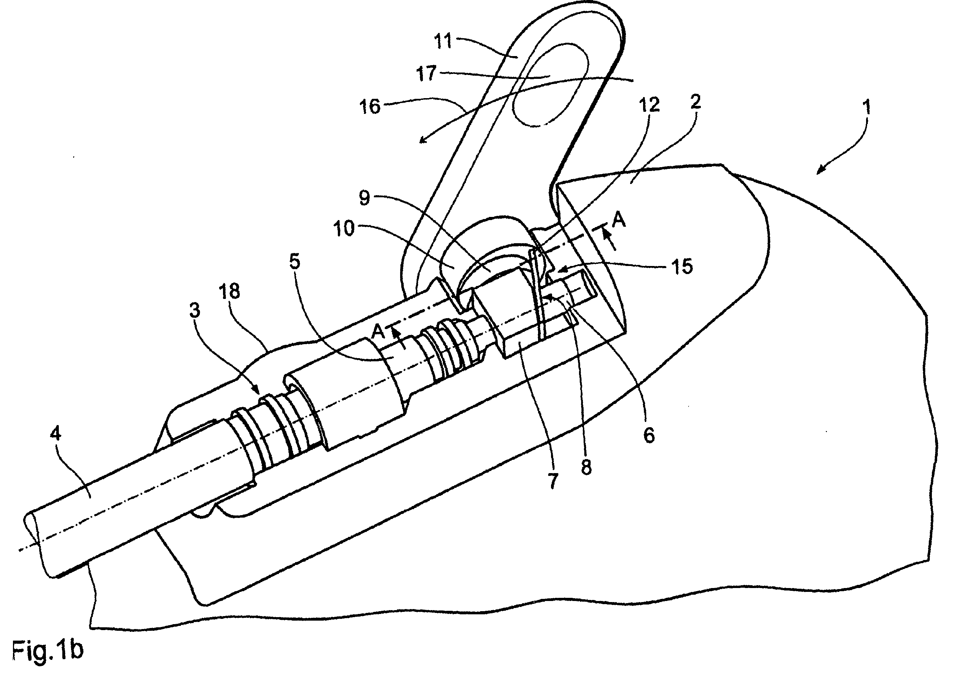

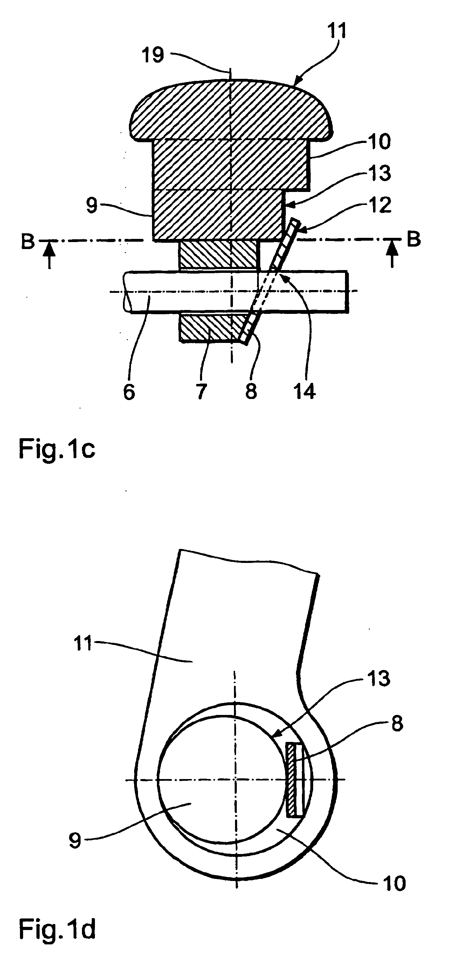

Der in den Figuren 1a und 1b gezeigte Herzschrittmacher 1 weist einen Header 2 auf, in welchem eine Anschlußbuchse 3 angeordnet ist, über welche eine koaxiale Elektrodenleitung 4 mittels eines standardisierten IS-1-Steckers 5 mit dem Herzschrittmacher 1 elektrisch verbunden ist.

Der differente Pol 6 des in die Anschlußbuchse 3 eingesteckten Steckers 5 am proximalen Ende der koaxialen Elektrodenleitung 4 befindet sich in einer Steckeraufnahme 7, welche innerhalb der Anschlußbuchse 3 vorgesehen ist. Die Steckeraufnahme 7 trägt an ihrer der Einstecköffnung für den Stecker 5 gegenüberliegenden Seite ein Kontaktelement 8. Das Kontaktelement 8 ist zungenförmig ausgebildet und besteht aus einem Federblech. Die Befestigung des Kontaktelements 8 ist derart einseitig an der Wandung der Steckeraufnahme 7, bevorzugt durch Verschweißen, vorgenommen, daß sich die Achse des Kontaktelements im wesentlichen quer zur Längsachse der Steckeraufnahme 7 bzw. des Steckers 5 erstreckt.

An der in Erstreckungsrichtung des Kontaktelements 8 angeordneten Wandung der Steckeraufnahme ist eine als Exzenter ausgebildete Spannocke 9 angeordnet, welche über einen in der Wandung des Headers 2 gelagerten Lagerzapfen 10 mit einem Handhebel 11 verbunden ist. Der Durchmesser der Spannocke 9 ist derart gewählt, daß sich das freie Ende 12 des Kontaktelements 8 auf ihrer Flanke (vergleiche die Position 13 in Figur 1d) abstützt.

In dem federnd an der Steckeraufnahme 7 befestigten Kontaktelement 8 ist eine Bohrung (vergleiche die Position 14 in Figur 1c) vorgesehen, durch welche sich der differente Pol 6 bewegt, wenn der Stecker 5 zum Verbinden der koaxialen Elektrodenleitung 4 mit dem Herzschrittmacher 1 in die Anschlußbuchse 3 eingesteckt wird.

Das Kontaktelement 8 und die Spannocke 9 bilden die Elemente einer Verriegelungseinrichtung 15, welche durch Schwenken des Handhebels 11 in Richtung des Pfeils 16 aktivierbar ist und von einem geöffneten Zustand, in welchem der Stecker 5 in die Anschlußbuchse 3 geschoben bzw. aus dieser abgezogen werden kann, in den geschlossen Zustand verbracht wird, in welchem ein in der Anschlußbuchse 3 befindlicher Stecker 5 gegen ein unerwünschtes, durch eine in axialer Richtung wirksame Zugkraftkomponente bewirktes Herausgleiten aus der Anschlußbuchse 3 gesichert ist.

Befindet sich die Verriegelungseinrichtung 15 im geschlossenen Zustand, wird durch das Zusammenwirken der Exzenternocke mit dem Kontaktelement 8 zwischen dem differenten Pol 6 und dem Kontaktelement 8 eine Klemmkraft erzeugt, welche den differenten Pol 6 des Steckers 5 in der Steckeraufnahme 7 fixiert.

Mit 17 und 18 sind als Ausnehmung bzw. als Ansatz ausgebildete Rastmittel an der Außenseite des Headers 2 und an der Innenseite des Handhebels 11 bezeichnet. Die Rastmittel gelangen in Wirkungseingriff, wenn der Handhebel in Richtung des Pfeils 16 zum Schließen der Verriegelungseinrichtung 15 geschwenkt wird. Dadurch wird auf einfache Weise sichergestellt, daß der Handhebel in dieser Stellung gegen eine unerwünschte Rückbewegung fixiert ist.

In den Figuren 1c und 1d ist der Aufbau und die Wirkungsweise der aus dem Kontaktelement 8 und der Exzenternocke 9 gebildeten Verriegelungseinrichtung zu ersehen.

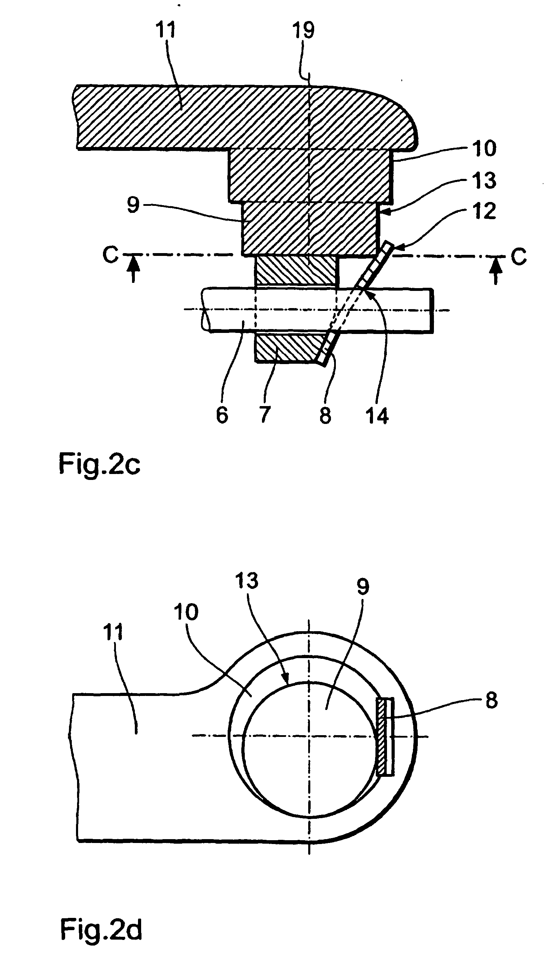

Das an der Steckeraufnahme 7 befestigte Kontaktelement 8 weist eine Bohrung 14 auf, welche bei geöffneter Verriegelungseinrichtung einen freien Querschnitt ausreichender Größe aufweist, durch welchen sich der differente Pol 6 des Stekkers bewegen kann, wenn dieser in die Anschlußbuchse geschoben wird. Nach Schwenken des durch den Lagerzapfen 10 im Header gelagerten Handhebels 11 um die Achse 19, welche sich quer zur Längachse der Anschlußbuchse erstreckt, wird das sich an der Flanke 13 der Exzenternocke abstützende Kontaktelement 8 reversibel verformt. Diese Verformung (vergleiche die Darstellung gemäß der Figuren 2c und 2d) bewirkt eine kraftschlüssige Verbindung zwischen dem Kontaktelement 8 und dem in der Steckeraufnahme 7 befindlichen differenten Pol 6 des Steckers.

Der jeweils vorhandene Verriegelungszustand der Verriegelungseinrichtung 15 ist auf einfache Weise an der Stellung des Handhebels 11 zu erkennen. Steht der Handhebel im wesentlichen quer zur Längsachse der Anschlußbuchse 3, ist die Verriegelungseinrichtung geöffnet. Ein sich parallel zur Längsachse der Anschlußbuchse erstreckender Handhebel weist auf eine geschlossene Verriegelungseinrichtung hin.

Bedingt durch die Länge des Handhebels 11 ist zur Erzeugung der den Stecker in der Anschlußbuchse 3 fixierenden Klemmkraft zwischen Kontaktelement 8 und differentem Pol 6 erforderliche Kraftaufwand relativ gering, was für den praktischen Anwendungsfall, beispielsweise unter Operationsbedingungen in einer medizinischen Einrichtung, von Vorteil ist.

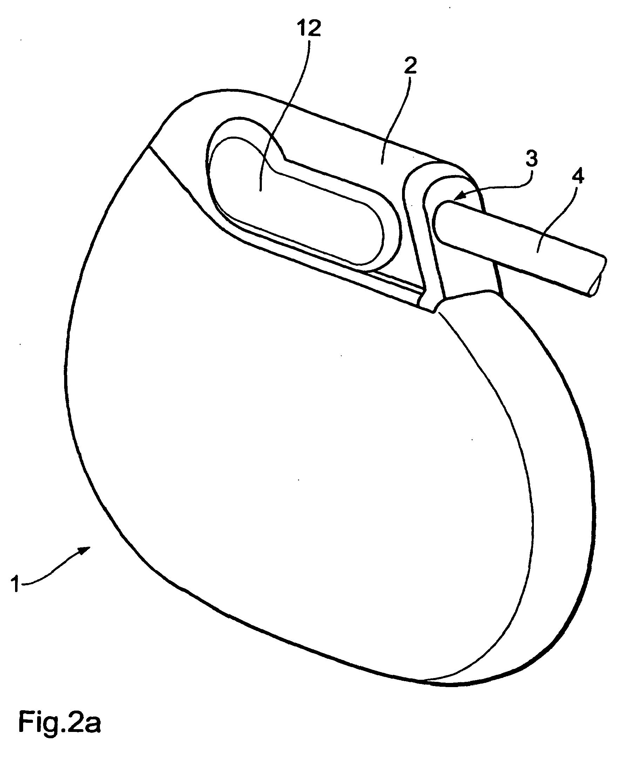

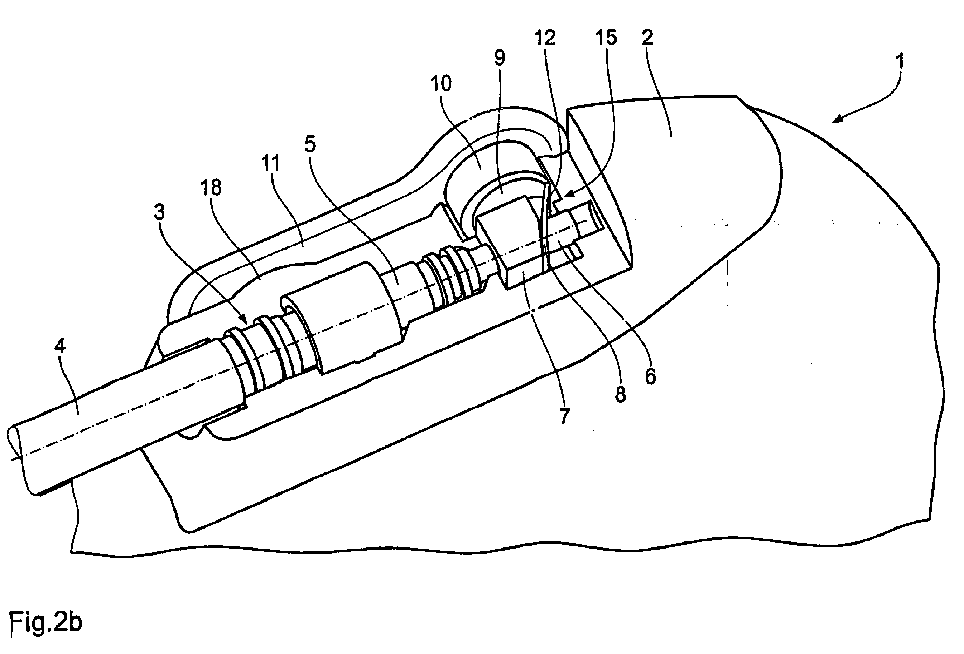

Die Figuren 2a, 2b, 2c und 2d zeigen die Position der Elemente 8 und 9 der Verriegelungseinrichtung 15, wenn sich diese in geschlossenem Zustand befindet.

Der Handhebel 11 ist in Richtung der koaxialen Elektrodenleitung 4 (vergleiche die Position 16 in Figur 1) geklappt, so daß sich das an der Flanke 13 der Exzenternocke 9 abstützende Ende 12 des federnden Kontaktelements 8 in der Zeichnungsebene der jeweiligen Figur nach rechts bewegt, wodurch sich der durch Projektion auf die Querschnittsfläche des differenten Pols 6 bestimmte, freie Durchmesser der Bohrung 14 verringert. Dies bewirkt durch Klemmwirkung eine ausreichend große Haltekraft zwischen differentem Pol 6 und dem Kontaktelement 8 bei einer axialen Zugbelastung der Elektrodenleitung 4. Die entsprechende Zugkraft wird über das Kontaktelement 8 über die Steckeraufnahme 7 bzw. die Exzenternocke 9 in die Wandung des Headers 2 eingeleitet.

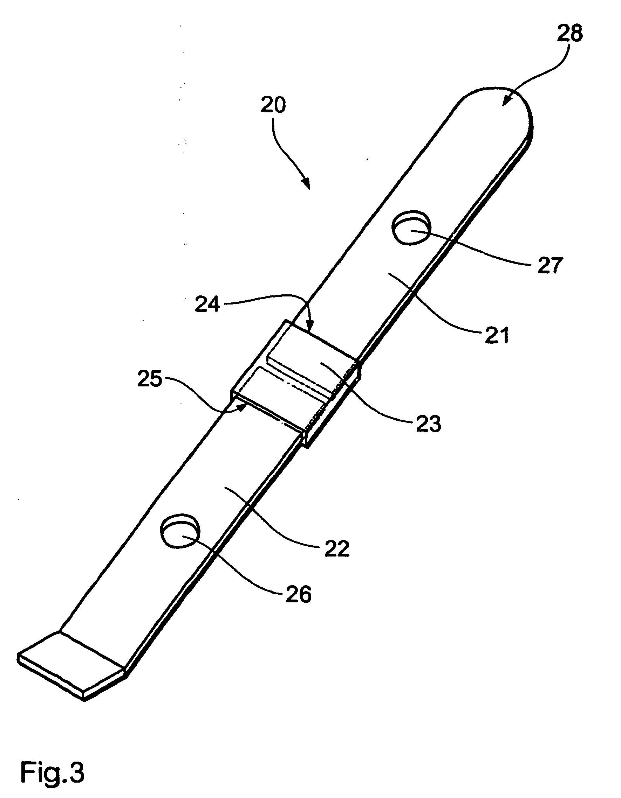

Figur 3 zeigt ein aus einem metallischen Federmaterial hergestelltes, Kontaktelement 20, welches aus zwei, gegeneinander elektrisch isoliert miteinander verbundenen Teilstücken 21 und 22 besteht. Zur Verbindung der beiden Teilstücke ist ein Koppelstück 23 mit zwei Einstecktaschen 24 und 25 vorgesehen, in welche jeweils ein Ende der Teilstücke 21 und 22 derart eingepreßt und/oder eingeklebt sind, daß sich die Feder- und Biegeeigenschaften des gesamten Kontaktelements 20 von einem einstückig ausgebildeten Kontaktelement im wesentlichen nicht unterscheiden. Die Bohrungen 26 und 27 können jeweils einen differenten Pol des Steckers einer koaxialen Elektrodenleitung aufnehmen, so daß durch eine Drehbewegung einer an dem freien Ende 28 des Kontaktelements 20 angreifenden Spannockes (vergleiche die Position 9 in den Figuren 1c und 2c) gleichzeitig beide Stecker in der entsprechenden Anschlußvorrichtung gegen ein unerwünschtes Herausgleiten gesichert werden können.

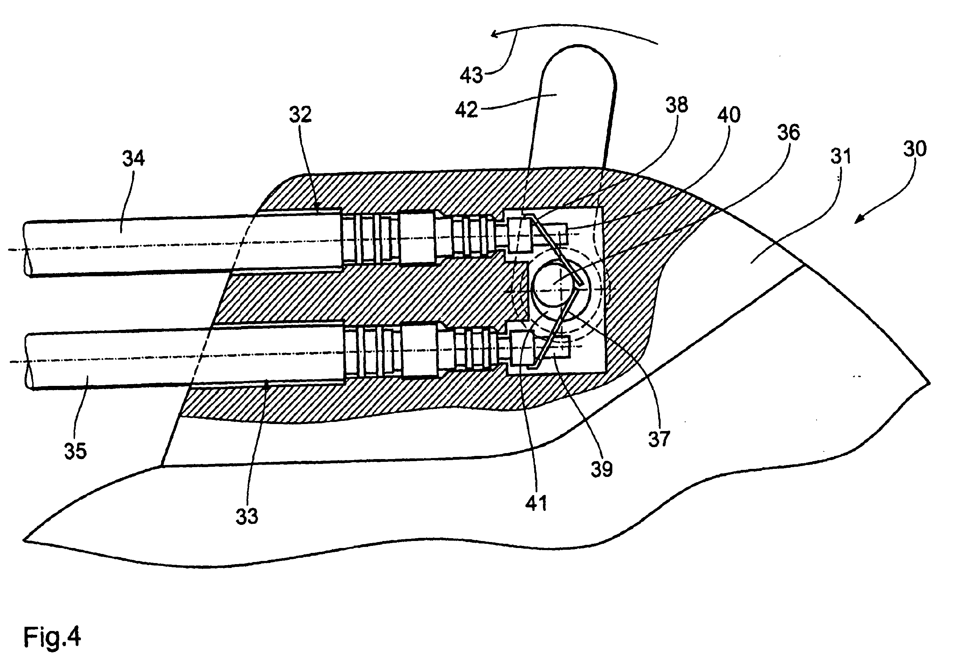

Der in Figur 4 gezeigte Herzschrittmacher 30 weist einen Header 31 mit zwei Anschlußbuchsen 32 und 33 für jeweils einen Stecker einer koaxialen Elektrodenleitung 34, 35 auf.

Der als Exzenternocke ausgebildete Spannocke 36 befindet sich mit zwei, im wesentlichen spiegelsymmetrisch zueinander angeordneten Kontaktelementen 37 und 38 in Wirkkontakt. Die differenten Pole 39, 40 der Stecker sind durch eine (nicht bezeichnete) Bohrung in dem jeweiligen Kontaktelement geführt und werden innerhalb dieser Bohrung durch Klemmwirkung gehalten, wenn der mit der Exzenternocke 36 über den Lagerzapfen 41 verbundene Handhebel 42 in Richtung des Pfeils 43 geschwenkt wird.

Durch die in Figur 4 im entriegelten Zustand dargestellte Konstruktion können in vorteilhafter Weise mit einer Schwenkbewegung des Handhebels 42 gleichzeitig zwei Stecker gegen unbeabsichtigtes Lösen aus den Anschlußbuchsen 32, 33 gesichert werden.

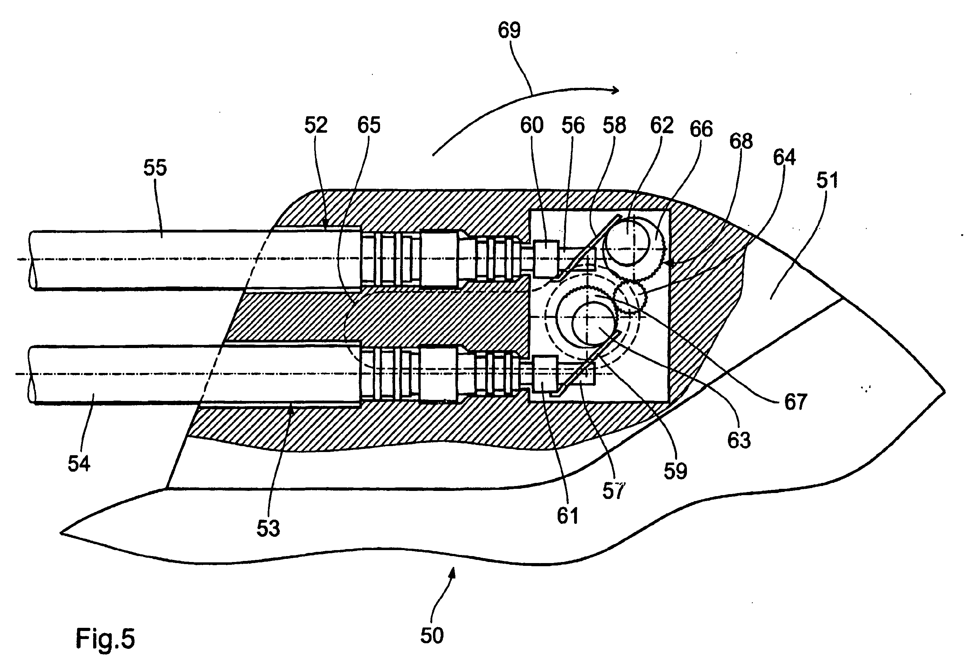

Für den in Figur 5 gezeigten Herzschrittmacher 5 ist ein Header 51 mit zwei Anschlußbuchsen 52 und 53 vorgesehen, in welchen sich jeweils der Stecker einer koaxiale Elektrodenleitung 54, 55 befindet. Die differenten Pole 56, 57 der Stecker sind in ihrer durch die jeweilige Verriegelungseinrichtung fixierten Position dargestellt.

Zur Verbindung der elektrischen Kontaktelemente 58 und 59 mit der jeweiligen Steckeraufnahme 60 und 61 ist eine Schweißverbindung vorgesehen. Die Kontaktelemente erstrecken sich parallel zueinander. Zur Bildung der die differenten Pole 56, 57 fixierenden Verriegelungseinrichtungen ist jedem der Kontaktelemente 58, 59 eine Exzenternocke 62, 63 zugeordnet, auf welchen sich die freien Enden der Kontaktelemente 58, 59 abstützen.

Die Exzenternocke 62 wird mittelbar über ein Ritzel 64 angetrieben, wenn sich die Exzenternocke 63 beim Schwenken des Handhebels 65 dreht. Zur Bildung des entsprechenden Getriebes tragen die Lagerzapfen 66, 67 der Exzenternocken 62, 63 eine Außenverzahnung 68. Zur Sicherung der Getriebefunktion zur Kraftübertragung durch die Bewegung des Handhebels 65 ist es ausreichend, wenn sich die Außenverzahnung über den halben Umfang der Lagerzapfen 66, 67 erstreckt.

Beim Bewegen des Handhebels 65 in Richtung des Pfeils 69 werden die Kontaktelemente 58, 59 von dem Druck der jeweiligen Exzenternocke 62, 63 entlastet. Dabei schwenkt das Kontaktelement 58 bzw. 59 mit seinem freien Ende in der Zeichnungsebene geringfügig nach unten bzw. nach oben, wodurch die kraftschlüssige Verbindung zwischen den Kontaktelementen 58, 58 und den differenten Polen 56, 57 aufgehoben wird und der jeweilige Stecker der Elektrodenleitungen 54, 55 bequem aus der Anschlußbuchse 52, 53 gezogen werden kann. Das Entsperren der beiden Verriegelungseinrichtungen gleichzeitig durch eine einzige Hebeldrehung um etwa 90°, wobei der entsperrte Zustand der Verriegelungseinrichtungen durch die im wesentlichen senkrechte Stellung des Handhebels 65 an der Außenwandung des Headers 51 eindeutig zu erkennen ist.

Die Fixierung der in den Figuren 4 und 5 gezeigten Handhebel 42 und 65 gegen eine unerwünschte Rückbewegung aus einer die Verriegelungseinrichtungen sperrenden Stellung erfolgt durch (nicht dargestellte) Rastmittel in der Art und Weise, wie in den Ausführungen zu Figur 1b bereits erläutert.

Das Fixieren beider Stecker in den Anschlußbuchsen 32, 33 bzw. 52, 53 durch eine Schwenkbewegung des Handhebels 42 bzw. 65 mit relativ geringem Krafteinsatz ist für die praktische Anwendung, beispielsweise unter Operationsbedingungen in einer medizinischen Einrichtung, von besonderem Vorteil.

Aus Gründen der Übersichtlichkeit wurde in den Figuren 1b, 2b, 4 und 5 von einer Darstellung der elektrischen Leitungsverbindung von zu den elektronischen Baugruppen des jeweilgen Herzschrittmachers 1, 30 und 50 abgesehen.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Implantierbares elektronisches Gerät (1, 30, 50), insbesondere Herzschrittmacher, mit mindestens einer im Header (2, 31, 51) angeordneten, im wesentlichen buchsenförmigen Anschlußvorrichtung (3, 32, 33, 52, 53) für einen an dem proximalen Ende einer Elektrodenleitung (4, 34, 35, 54, 55) angeordneten Stecker, mit einer ein Kontaktelement (8, 20, 37, 38, 58, 59) aufweisenden Steckeraufnahme (7, 60, 61) für den mindestens einen differenten Pol (6, 39, 40, 56, 57) des Steckers, wobei an der Anschlußvorrichtung eine den Sitz des Steckers bei einer in axialer Richtung wirksamen Zugkraft sichernde, durch einen an der Außenseite des Headers befindlichen Handhebel (11) bedienbare, Verriegelungseinrichtung (15) vorgesehen ist, **dadurch gekennzeichnet, daß** die Verriegelungseinrichtung (15) durch das Kontaktelement (8, 20, 37, 38, 58, 59) und eine in der Anschlußvorrichtung (3, 32, 33, 52, 53) exzentrisch gelagerte Spannocke (9, 36, 62, 63) gebildet ist, wobei das federnd angeordnete Kontaktelement
- den differenten Pol (6, 39, 40, 56, 57) des Steckers umgreift und
- derart mit der Spannocke (9, 36, 62, 63) in Wirkungseingriff steht,
daß bei einer Drehbewegung der Spannocke eine Veränderung der Lage und/oder der Form des Kontaktelements relativ zu dem differenten Pol des Steckers eine kraftschlüssigen Verbindung zwischen dem differenten Pol und dem Kontaktelement zwecks Fixation des Steckers und Herstellung eines galvanischen Kontakts erzwungen wird.

2. Elektronisches Gerät nach Anspruch 1, **dadurch gekennzeichnet, daß** eine scheibenförmige Spannocke (9, 36, 62, 63) vorgesehen ist.

3. Elektronisches Gerät nach Anspruch 2, **dadurch gekennzeichnet, daß** die Spannocke (9, 36, 62, 63) im wesentlichen die Form einer Kreisscheibe aufweist.

4. Elektronisches Gerät nach Anspruch 2 oder 3, **dadurch gekennzeichnet, daß** sich die Drehachse der Spannocke (9, 36, 62, 63) quer zur Längsachse des Steckers erstreckt.

5. Elektronisches Gerät nach einem der vorhergehenden Ansprüche, **dadurch gekennzeichnet, daß** das Kontaktelement (8, 20, 37, 38, 58, 59) als zungenförmiges, einseitig, bevorzugt an der Anschlußvorrichtung (3, 32, 33, 52, 53), befestigtes Federblech ausgebildet ist und sich im wesentlichen quer zur Längsachse des Steckers erstreckt.

6. Elektronisches Gerät nach Anspruch 5, **dadurch gekennzeichnet, daß** das Kontaktelement (8, 20, 37, 38, 58, 59) eine Bohrung aufweist, durch welche der differente Pol eines in der Steckeraufnahme befindlichen Stekkers geführt ist.

7. Elektronisches Gerät nach einem der vorhergehenden Ansprüche, **dadurch gekennzeichnet, daß** sich das Kontaktelement (8, 37, 38, 58, 59) mit seinem freien Ende auf der Kurvenbahn der exzentrisch gelagerten Nocke derart abstützt, daß sich bei Verdrehen des Spannockes durch Schwenken des Kontaktelements (8, 37, 38, 58, 59) der beim Einstecken des Steckers für den differenten Pol erforderliche freie Durchmesser der Bohrung verringert und der differente Pol (6, 39, 40, 56, 57) durch eine Klemmkraft an dem Kontaktelement(8, 37, 38, 58, 59) fixiert wird.

8. Elektronisches Gerät nach Anspruch 7, **dadurch gekennzeichnet, daß** das Kontaktelement (8, 37, 38, 58, 59) zum Erzeugen der Klemmwirkung durch reversibles Verformen mittels der Spannocke (9, 36, 62, 63) aus einem entspannten Zustand in einen gespannten Zustand überführt wird.

9. Elektronisches Gerät nach Anspruch 6, **dadurch gekennzeichnet, daß** für den Spannocke ein Drehbereich von bevorzugt 120° vorgesehen ist.

10. Elektronisches Gerät nach einem der vorhergehenden Ansprüche, **dadurch gekennzeichnet, daß** an der Außenseite des Headers (2, 31, 51) und der dem Header zugewandten Seite des Handhebels (11) jeweils ein Rastmittel (17, 18) vorgesehen ist, welche sich bei geschlossener Verriegelungseinrichtung (15) kraft- und/oder formschlüssig in Eingriff befinden und den Handhebel gegen eine unerwünschte, zum Öffnen der Verriegelungseinrichtung führende Rückbewegung fixieren.

11. Elektronisches Gerät nach Anspruch 10, **dadurch gekennzeichnet, daß** die Rastmittel als Ansatz (18) und eine den Ansatz aufnehmende Auskehlung (17) entsprechender Größe ausgebildet sind.

12. Elektronisches Gerät nach Anspruch 1, **dadurch gekennzeichnet, daß** das Kontaktelement (20) zweiteilig ausgebildet ist, wobei in jedem der Teilstücke (21, 22) eine Bohrung (26, 27) zur Aufnahme des differenten Pols eines jeweils in einer Anschlußvorrichtung(3,32,33, 52, 53) befindlichen - Steckers vorgesehen ist und die beiden Teilstücke (21,22) durch einen Verbinder (25) aus Kunststoff miteinander gekoppelt sind.

13. Elektronisches Gerät nach einem der vorhergehenden Ansprüche, **dadurch gekennzeichnet, daß** die Spannocke (36) zwischen zwei im wesentlichen gleichartig ausgebildeten, vertikal übereinander positionierten Anschlußvorrichtungen (32, 33) angeordnet ist, deren Kontaktelemente (38, 39) sich jeweils bei im wesentlichen spiegelsymmetrischer Anordnung zwecks Bildung einer Verriegelungsvorrichtung (15) auf dem Spannocke abstützen.

14. Elektronisches Gerät nach Anspruch 13, **dadurch gekennzeichnet, daß** für jede Anschlußvorrichtung (52, 53) eine gesonderte Spannocke (62, 63) vorgesehen ist, wobei die erste Spannnocke (63) mit einem Handhebel (65) verbunden ist und die Drehbewegung der ersten Spannocke (63) von diesem kraft- und formschlüssig auf die zweite Spannocke (62) übertragen wird.

15. Elektronisches Gerät nach Anspruch 14, **dadurch gekennzeichnet, daß** die Spannocken (62, 63) je einen Lagerzapfen (66, 67) aufweisen, welche zur Übertragung der Drehbewegung, zumindest an einem Abschnitt ihrer Peripherie, eine Außenverzahnung (68) tragen.

16. Elektronisches Gerät nach Anspruch 15, **dadurch gekennzeichnet, daß** zur Übertragung der Drehbewegung eine mittelbare Verbindung zwischen den Spannocken (62, 63) vorgesehen ist.

17. Elektronisches Gerät nach Anspruch 16, **dadurch gekennzeichnet, daß** die die Außenverzahnung (68) der Lagerzapfen (66, 67) der Spannocken (62, 63) direkt oder über ein Ritzel (64) in Eingriff steht.

## Claims

1. An implantable electronic device (1, 30, 50), particularly a pacemaker, having at least one essentially bush-shaped connection unit (3, 32, 33, 52, 53), which is positioned in the header (2, 31, 51), for a plug positioned on the proximal end of an electrode line (4, 34, 35, 54, 55), having a plug receptacle (7, 60, 61), which has a contact element (8, 20, 37, 38, 58, 59) for the at least one different pole (6, 39, 40, 56, 57) of the plug, a locking unit (15), which secures the seat of the plug in the event of a tensile force in the axial direction and is operable by a hand lever (11) located on the outside of the header, being provided on the connection device,
**characterized in that** the locking unit (15) is formed by the contact element (8, 20, 37, 38, 58, 59) and a clamping cam (9, 36, 62, 63), which is eccentrically mounted in the connection device (3, 32, 33, 52, 53), the elastically arranged contact element
- enclosing the different pole (6, 39, 40, 56, 57) of the plug and
- being mechanically engaged with the clamping cam (9, 36, 62, 63) in such a way
that, in the event of a rotational movement of the clamping cam, a change of the position and/or the shape of the contact element in relation to the different pole of the plug forces a non-positive connection between the different pole and the contact element for the purpose of fixing the plug and producing a galvanic contact.

2. The electronic device according to Claim 1,
**characterized in that** a disk-shaped clamping cam (9, 36, 62, 63) is provided.

3. The electronic device according to Claim 2,
**characterized in that** the clamping cam (9, 36, 62, 63) essentially has the form of a circular disk.

4. The electronic device according to Claim 2 or 3,
**characterized in that** the rotational axis of the clamping cam (9, 36, 62, 63) extends transversely to the longitudinal axis of the plug.

5. The electronic device according to one of the preceding claims,
**characterized in that** the contact element (8, 20, 37, 38, 58, 59) is implemented as a tongue-shaped spring steel sheet, which is attached on one side, preferably to the connection device (3, 32, 33, 52, 53), and extends essentially transversely to the longitudinal axis of the plug.

6. The electronic device according to Claim 5,
**characterized in that** the contact element (8, 20, 37, 38, 58, 59) has a hole, through which the different pole of a plug located in the plug receptacle is guided.

7. The electronic device according to one of the preceding claims,
**characterized in that** the free end of the contact element (8, 37, 38, 58, 59) is supported on the curved path of the eccentrically mounted cam in such a way that as the clamping cam rotates due to pivoting of the contact element (8, 37, 38, 58, 59), the free diameter of the hole required for the different pole upon insertion of the plug is reduced and the different pole (6, 39, 40, 56, 57) is fixed on the contact element (8, 37, 38, 58, 59) by a clamping force.

8. The electronic device according to Claim 7,
**characterized in that** the contact element (8, 37, 38, 58, 59) is transferred from a relaxed state into a clamped state to generate the clamping force by reversible deformation using the clamping cam (9, 36, 62, 63).

9. The electronic device according to Claim 6,
**characterized in that** a rotational range of 120° is preferably provided for the clamping cam.

10. The electronic device according to one of the preceding claims,
**characterized in that** catch means (17, 18) is provided both on the outside of the header (2, 31, 51) and on the side of the hand lever (11) facing toward the header, which are non-positively and/or positively engaged when the locking unit (15) is closed and fix the hand lever against an undesired return movement, which would result in opening of the locking unit.

11. The electronic device according to Claim 10,
**characterized in that** the catch means are implemented as a projection (18) and a recess (17) of corresponding size, which receives the projection.

12. The electronic device according to Claim 1,
**characterized in that** the contact element (20) is implemented in two parts, a hole (26, 27) being provided in each of the parts (21, 22) to receive the different pole of a plug located in a connection device (3, 32, 33, 52, 53), and the two parts (21, 22) being coupled to one another by a connector (25) made of plastic.

13. The electronic device according to one of the preceding claims,
**characterized in that** the clamping cam (36) is positioned between two essentially identically implemented connection units (32, 33), which are positioned vertically one over another, whose contact elements (38, 39) are each supported on the clamping cam if they are positioned essentially mirror-symmetrically, for the purpose of forming a locking unit (15).

14. The electronic device according to Claim 13,
**characterized in that** a separate clamping cam (62, 63) is provided for each connection unit (52, 53), the first clamping cam (63) being connected to a hand lever (65) and the rotational movement of the first clamping cam (63) being transmitted non-positively and positively therefrom to the second clamping cam (62).

15. The electronic device according to Claim 14,
**characterized in that** the clamping cams (62, 63) each have a bearing pin (66, 67), which carry external teeth (68) on at least a section of their circumference to transmit the rotational movement.

16. The electronic device according to Claim 15,
**characterized in that** an indirect connection is provided between the clamping cams (62, 63) to transmit the rotational movement.

17. The electronic device according to Claim 16,
**characterized in that** the external teeth (68) of the bearing pins (66, 67) of the clamping cams (62, 63) are engaged directly or via a pinion (64).

## Revendications

1. Appareil (1, 30, 50) électronique implantable, en particulier pacemaker, comprenant au moins un dispositif de raccordement (3, 32, 33, 52, 53) sensiblement en forme de douille et disposé dans le header (2, 31, 51) pour une prise mâle disposée sur l'extrémité proximale d'une ligne d'électrodes (4, 34, 35, 54, 55), un logement de prise (7, 60, 61) présentant un élément de contact (8, 20, 37, 38, 58, 59) pour le au moins un pôle (6, 39, 40, 56, 57) différent de la prise, un dispositif de verrouillage (15) bloquant le siège de la prise avec une force de traction efficace dans le sens axial et pouvant être manoeuvré par un levier manuel (11) situé sur le côté extérieur du Header étant prévu sur le dispositif de raccordement, **caractérisé en ce que** le dispositif de verrouillage (15) est formé par l'élément de contact (8, 20, 37, 38, 58, 59) et une came de serrage (9, 36, 62, 63) logée de façon excentrée dans le dispositif de raccordement (3, 32, 33, 52, 53), l'élément de contact disposé de façon élastique
- entourant le pôle (6, 39, 40, 56, 57) différent de la prise et
- étant en prise active de ce fait avec la came de serrage (9, 36, 62, 63),
**en ce que**, lors d'un mouvement de rotation de la came de serrage, une variation de la position et/ou de la forme de l'élément de contact par rapport au pôle différent de la prise est obtenue par force par une liaison d'adhérence entre le pôle différent et l'élément de contact pour la fixation de la prise et l'établissement d'un contact galvanique.

2. Appareil électronique selon la revendication 1, **caractérisé en ce qu'**une came de serrage (9, 36, 62, 63) en forme de disque est prévue.

3. Prise électronique selon la revendication 2, **caractérisé en ce que** la came de serrage (9, 36, 62, 63) présente sensiblement la forme d'un disque circulaire.

4. Appareil électronique selon la revendication 2 ou 3, **caractérisé en ce que** l'axe de rotation de la came de serrage (9, 36, 62, 63) s'étend transversalement à l'axe longitudinal de la prise.

5. Appareil électronique selon l'une quelconque des revendications précédentes, **caractérisé en ce que** l'élément de contact (8, 20, 37, 39, 58, 59) est réalisé sous la forme d'une tôle à ressort en forme de lame, fixée sur un côté, de préférence sur le dispositif de raccordement (3, 32, 33, 52, 53), et s'étend sensiblement transversalement à l'axe longitudinal de la prise.

6. Appareil électronique selon la revendication 5, **caractérisé en ce que** l'élément de contact (8, 20, 37, 38, 58, 59) présente un alésage à travers lequel le pôle différent d'une prise se trouvant dans le logement de prise est guidé.

7. Appareil électronique selon l'une quelconque des revendications précédentes, **caractérisé en ce que** l'élément de contact (8, 20, 37, 38, 58, 59) s'appuie par son extrémité libre sur la voie incurvée de la came logée de façon excentrée, **en ce que**, lors de la rotation de la came de serrage, le diamètre libre de l'alésage, nécessaire lors de l'emboîtement de la prise pour le pôle différent, se réduit par le basculement de l'élément de contact (8, 37, 38, 58, 59) et le pôle différent (6, 39, 40, 56, 57) est fixé par une force de serrage sur l'élément de contact (8, 37, 38, 58, 59).

8. Appareil électronique selon la revendication 7, **caractérisé en ce que** l'élément de contact (8, 37, 38, 58, 59) est transféré d'un état desserré dans un état serré pour générer l'effet de blocage par la déformation réversible au moyen de la came de serrage (9, 36, 62, 63).

9. Appareil électronique selon la revendication 6, **caractérisé en ce qu'**une plage de rotation de préférence de 120° est prévue pour la came de serrage.

10. Appareil électronique selon l'une quelconque des revendications précédentes, **caractérisé en ce que** sur le côté extérieur du Header (2, 31, 51) et le côté, tourné vers le Header, du levier manuel (11) est prévu respectivement un moyen de blocage (17, 18), lesquels se trouvent en prise par adhérence et/ou par complémentarité de forme lorsque le dispositif de verrouillage (15) est fermé et fixent le levier manuel contre un mouvement de recul inopportun et aboutissant à l'ouverture du dispositif de verrouillage.

11. Appareil électronique selon la revendication 10, **caractérisé en ce que** les moyens de blocage sont conçus comme un rebord (18) et une gorge recevant le rebord (17) de grandeur appropriée.

12. Appareil électronique selon la revendication 1, **caractérisé en ce que** l'élément de contact (20) est conçu en deux parties, un alésage (26, 27) pour le logement du pôle différent d'une prise se trouvant respectivement dans un dispositif de raccordement (3, 32, 33, 52, 53) étant prévu dans chacun des tronçons (21, 22) et les deux tronçons (21, 22) étant couplés entre eux par un connecteur (25) en plastique.

13. Appareil électronique selon l'une quelconque des revendications précédentes, **caractérisé en ce que** la came de serrage (36) est disposée entre deux dispositifs de raccordement (32, 33) réalisés sensiblement de la même façon et positionnés verticalement l'un au-dessus de l'autre, dont les éléments de contact (38, 39) s'appuient sur la came de serrage respectivement avec un agencement sensiblement symétrique pour former un dispositif de verrouillage (15).

14. Appareil électronique selon la revendication 13, **caractérisé en ce qu'**une came de serrage (62, 63) séparée est prévue pour chaque dispositif de raccordement (52, 53), la première came de serrage (63) étant reliée à un levier manuel (65) et le mouvement de rotation de la première came de serrage (63) étant transmis de celui-ci par adhérence et complémentarité de forme à la seconde came de serrage (62).

15. Appareil électronique selon la revendication 14, **caractérisé en ce que** les cames de serrage (62, 63) présentent respectivement un tourillon (66, 67), qui porte une denture extérieure (68) pour la transmission du mouvement de rotation, au moins sur une partie de sa périphérie.

16. Appareil électronique selon la revendication 15, **caractérisé en ce qu'**une liaison indirecte entre les cames de serrage (62, 63) est prévue pour la transmission du mouvement de rotation.

17. Appareil électronique selon la revendication 16, **caractérisé en ce que** la toiture extérieure (68) des tourillons (66, 67) des cames de serrage (62, 63) est en prise directe ou au moyen d'un pignon (64).
